# EUROPÄISCHE PATENTSCHRIFT

(11) **EP 1 140 789 B1**
(45) Veröffentlichungstag und Bekanntmachung des Hinweises auf die Patenterteilung: **11.06.2003**
(21) Anmeldenummer: 99973013.8
(22) Anmeldetag: 25.11.1999
(51) Int. Cl.: C07C 213/10, C07C 213/04

(54) **VERFAHREN ZUR REINIGUNG VON TRIETHANOLAMIN**
METHOD FOR PURIFYING TRIETHANOLAMINE
PROCEDE DE PURIFICATION DE TRIETHANOLAMINE

(30) Priorität: 01.12.1998 DE 19855383
(43) Veröffentlichungstag der Anmeldung: 10.10.2001
(73) Patentinhaber: BASF AKTIENGESELLSCHAFT, 67056 Ludwigshafen (DE)
(72) Erfinder: RUIDER, Günther, D-67157 Wachenheim (DE); ROSS, Karl-Heinz, D-67269 Grünstadt (DE)
(86) Internationale Anmeldenummer: EP9909138
(87) Internationale Veröffentlichungsnummer: WO00032553

(56) Entgegenhaltungen:
- EP-A- 0 004 015
- EP-A- 0 673 920

## Beschreibung

Die vorliegende Erfindung betrifft ein Verfahren zur Reinigung von Triethanolamin (TEA), hergestellt durch Umsetzung von wässrigem Ammoniak mit Ethylenoxid in flüssiger Phase unter Druck und bei erhöhter Temperatur.

Wichtige Einsatzgebiete von TEA sind beispielsweise Seifen, Waschmittel und Shampoos in der kosmetischen Industrie oder auch Dispergiermittel und Emulgiermittel.

Für diese Einsatzgebiete ist ein wasserklares, farbloses TEA, das diese Eigenschaft auch über längere Lagerzeit beibehält, erwünscht.

Bekannt ist, dass ein nach einer fraktionierenden Destillation eines TEA-Rohprodukts, das durch Umsetzung von wässrigem Ammoniak mit Ethylenoxid und Abdestillieren von Monoethanolamin (MEA) und Diethanolamin (DEA) gewonnen wurde, erhaltenes und zunächst farbloses reines TEA (Farbzahl: ca. 0 bis 20 APHA nach DIN-ISO 6271(= Hazen)), sich nach einer Lagerzeit von ca. 4 bis 6 Wochen, auch im geschlossenen Gebinde und unter Lichtausschluss, allmählich leicht rosa und schließlich, besonders leicht beim Stehen am Licht, gelb bis braun verfärben kann. Dieser Effekt wird durch Einwirkung von höheren Temperaturen beschleunigt. (Siehe z.B.: G.G. Smirnova et al., J. of Applied Chemistry of the USSR 61, S. 1508-9 (1988), und Chemical & Engineering News 1996, Sept. 16, Seite 42, mittlere Spalte).

Gemäß Chemical & Engineering News 1996, Sept. 16, Seite 42, zersetzt sich bei erhöhter Temperatur ein Mol TEA in ein Mol Ethanolamin und zwei Mol Acetaldehyd. Acetaldehyd kondensiert zu Crotonaldehyd, der wiederum mit Ethanolamin eine Schiff'sche Base bildet. Diese ungesättigte Schiff'sche Base führt unter 1,4-Polymerisation zu farbigen Produkten im TEA.

Zur Beurteilung der Farbqualität vom reinem TEA hat sich neben den zeitaufwendigen Lagerversuchen, bei denen die APHA-Farbzahl (nach DIN-ISO 6271) des TEAs in Abhängigkeit von der Lagerzeit gemessen wird, der sogenannte "Säureneutralisationstest" bewährt.

Dieser "Säureneutralisationstest" erlaubt die Beurteilung der farblichen Lagerstabilität von frisch hergestelltem TEA innerhalb weniger Minuten.

Der Test wird in den japanischen Dokumenten JP-A-62 019 558 (Derwent Abstract Nr. 87-067647/10) und JP-A-62 005 939 (Derwent Abstract Nr. 87-047397/07) beschrieben, wonach das TEA mit Essigsäure, Zitronensäure, Schwefelsäure, Salzsäure oder Phosphorsäure behandelt (neutralisiert) wird und danach die Extinktion der Absorptionsbanden bei 420 nm und 530 nm gemessen wird. Tritt während der Durchführung des Tests keine augenscheinliche Rosaverfärbung des TEAs auf und bleiben die gemessenen Werte für die Extinktion genügend klein, so ist das TEA farblich lagerstabil, bleibt also über einen Zeitraum von mehreren Monaten farblos.

In der Literatur sind verschiedene Verfahren zur Herstellung von reinem und farblosen bis wenig gefärbten TEA beschrieben.

EP-A-4015 beschreibt, dass Ethanolamine mit geringerer Verfärbung durch Zusatz von phosphoriger oder unterphosphoriger Säure während oder nach der Herstellung der Ethanolamine erhalten werden.

EP-A-36 152 und EP-A-4015 erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US-A-3 819 710 offenbart ein Verfahren zur Verbesserung der Farbqualität von Ethanolaminen durch Hydrierung der rohen Ethanolamine in Gegenwart ausgewählter Katalysatoren. Das Verfahren ist jedoch technisch aufwendig und führt nicht zu einer TEA-Ware, die über mehrere Monate farblos bleibt.

US-A-3 207 790 beschreibt ein Verfahren zur Verbesserung der Farbqualität von Alkanolaminen durch Zugabe eines Borhydrids eines Alkalimetalls.

US-A-3 742 059 und DE-A-22 25 015 beschreiben die Verbesserung der Farbqualität von Alkanolaminen durch den Zusatz eines Alkanolaminesters der Borsäure bzw. Alkali-/Erdalkalimetallboraten.

Die Anwesenheit eines Hilfsstoffes zur Stabilisierung von TEA ist jedoch in vielen wichtigen Anwendungsbereichen des TEAs unerwünscht.

Die nachträgliche Zugabe kleiner Mengen Ethylenoxid zu frisch destilliertem TEA führt nach US-A-4 673 762 ebenfalls zu einer Entfärbung und Farbstabilisierung. Die Methode erscheint jedoch aus toxikologischen Gründen bedenklich.

GB-A-1 062 730 beschreibt ein Verfahren zur Reinigung von Ethanolaminen durch Durchführung der Reindestillation in Gegenwart von Silikaten oder Aluminaten.

JP-A-62 019 558 (Derwent Abstract Nr. 87-067647/10) berichtet über die Herstellung von qualitativ gutem TEA durch Behandlung von rohem TEA mit anorganischen Oxiden bei 170 bis 250°C und anschließender Destillation in Abwesenheit von Sauerstoff.

Ähnliche Ergebnisse werden gemäß JP-A-62 005 939 (Derwent Abstract Nr. 87-047397/07) erzielt, wenn rohes TEA unter Luftausschluss 1 bis 10 h auf 170 bis 250°C erhitzt und dann im Vakuum destilliert wird.

SU-A-326 178 (Derwent Abstract Nr. 63384T-AE) beschreibt die Herstellung von TEA mit guter Farbqualität durch schonende Umsetzung von wasserfreiem Monoethanolamin (MEA) oder Diethanolamin (DEA) oder Mischungen beider Substanzen mit Ethylenoxid bei Temperaturen kleiner 50°C.

Ähnliche Ergebnisse werden gemäß SU-A-228 693 (Chem. Abstr. 70, 77305f (1969)) und GB-A-1 092 449 erzielt, wenn Ammoniak mit Ethylenoxid bei kleiner/gleich 35°C umgesetzt und die erhaltene Ethanolamin-Mischung unter Luftausschluss destilliert wird.

Vom wirtschaftlichen Gesichtspunkt sind solche Verfahren, bei denen die Umsetzungen mit Ethylenoxid bei niedrigen Temperaturen stattfinden, wegen der langen Verweilzeiten und der damit verbundenen niedrigen Raum-Zeit-Ausbeuten unrentabel.

Der vorliegenden Erfindung lag daher die Aufgabe zugrunde, ein alternatives wirtschaftliches Verfahren zur Herstellung von reinem, farblosen (APHA-Farbzahl kleiner/gleich 10) und farbstabilen Triethanolamin (TEA) aus wässrigem Ammoniak und Ethylenoxid aufzufinden.

Demgemäß wurde ein Verfahren zur Reinigung von Triethanolamin, hergestellt durch Umsetzung von wässrigem Ammoniak mit Ethylenoxid in flüssiger Phase unter Druck und bei erhöhter Temperatur gefunden, das dadurch gekennzeichnet ist, dass man vom Umsetzungsprodukt überschüssiges Ammoniak, Wasser und Monoethanolamin (MEA) abtrennt, das so erhaltene Rohprodukt mit Ethylenoxid bei Temperaturen von 110 bis 180°C umsetzt und anschließend in Gegenwart von phosphoriger oder unterphosphoriger Säure oder deren Verbindungen rektifiziert.

Das erfindungsgemäße Verfahren lässt sich wie folgt ausführen:
Zunächst wird, z.B. gemäß GB-A-760 215 oder EP-A-673 920, durch die Umsetzung von wässrigem Ammoniak mit Ethylenoxid in flüssiger Phase unter Druck und erhöhter Temperatur in einem mit einer Kühlung versehenen Reaktor ein Ethanolamin-Gemisch, enthaltend die Hauptkomponenten Monoethanolamin (MEA), Diethanolamion (DEA) und Triethanolamin (TEA), hergestellt. Bevorzugt wird hierbei das Verfahren gemäß der EP-A-673 920.

Die Reaktionstemperaturen betragen hierbei im allgemeinen 110 bis 160°C, bevorzugt 120 bis 150°C, und die Drücke 50 bis 120 bar (5 bis 12 MPa), bevorzugt 75 bis 100 bar (7,5 bis 10 MPa). Das molare Verhältnis von Ammoniak zu Ethylenoxid beträgt 1 : 1 bis 100 : 1, bevorzugt 3 : 1 bis 50 : 1, besonders bevorzugt 4 : 1 bis 15 : 1, und der Ammoniak wird als 60 bis 99,99 %ige, bevorzugt 70 bis 95 %ige, wässrige Lösung eingesetzt. Das eingesetzte Ethylenoxid kann als Gesamtmenge auf einmal oder in zwei bis zehn, bevorzugt zwei bis sechs, Teilmengen von jeweils 10 bis 70 Gew.-% (bezogen auf die Gesamtmenge) zugegeben werden.

Beispielsweise kann die Umsetzung von wässrigem Ammoniak mit Ethylenoxid gemäß dem Example 15 auf Seite 16 der GB-A-760 215 oder bevorzugt gemäß den beiden Versuchen Nr. 5 der Beispiele 1 und 2 der EP-A-673 920 durchgeführt werden.

Aus dem erhaltenen Produkt wird anschließend in an sich bekannter Weise der überschüssige Ammoniak zusammen mit einem Teil des Wassers unter Druck und danach das restliche Wasser bei vermindertem Druck abdestilliert.

Zurück bleibt ein im wesentlichen MEA, DEA und TEA enthaltendes Rohprodukt mit einem Wassergehalt kleiner 0,3 Gew.-%, bevorzugt kleiner 0,1 Gew.-%.

Nach der sich anschließenden destillativen Abtrennung des Monoethanolamins (MEA) bei vermindertem Druck verbleibt ein Rohprodukt bestehend aus DEA, TEA und geringe Mengen von Nebenkomponenten, wie beispielsweise (2-(2-Hydroxyethoxy)ethyl)-di-(2-hydroxyethyl)-amin, (2-(2-Hydroxyethoxy)ethyl)-(2-hydroxyethyl)-amin und N,N'-Di-(2-hydroxyethyl)-piperazin. Eine typischer Gehalt dieses Rohprodukts an DEA ist ca. 75 bis 80 Gew.-% und an TEA ist ca. 25 bis 20 Gew.-%.

Die Zusammensetzung dieses Rohprodukts kann je nach dem ursprünglich eingesetzten molaren Verhältnis von Ammoniak zu Ethylenoxid schwanken.

Anschließend wird dieses DEA und TEA enthaltende Rohprodukt, das einen Wassergehalt kleiner 0,3 Gew.-%, bevorzugt kleiner 0,1 Gew.-%, und einen Ammoniakgehalt kleiner 0,1 % Gew.-%, bevorzugt kleiner 0,01 Gew.-%, aufweist, mit 0,6 bis 1,2 Mol, bevorzugt 0,8 bis 1,1 Mol Ethylenoxid pro Grammatom an Stickstoff gebundenen Wasserstoff im Rohprodukt bei Temperaturen von 110 bis 180°C, bevorzugt 120°C bis 180°C, in flüssiger Phase umgesetzt. Diese Umsetzung erfolgt im allgemeinen wie in der GB-A-1 453 762 beschrieben. Bevorzugt erfolgt die Umsetzung in Rohrreaktoren und mehrstufig, wobei beispielsweise in einer ersten Reaktionsstufe bei Temperaturen von vorzugsweise 125 bis 165°C 50 bis 80 Gew.-% des eingesetzten Ethylenoxids, in einer zweiten Reaktionsstufe bei Temperaturen von vorzugsweise 150 bis 180°C die restliche Menge des eingesetzten Ethylenoxids umgesetzt wird und in einer dritten Umsetzungsstufe die Reaktion bei Temperaturen von 120 bis 150°C zu Ende geführt wird.

Die Umsetzung des DEA und TEA enthaltenden Rohprodukts mit Ethylenoxid kann beispielsweise wie in Example 12 auf Seite 4 der GB-A-1 453 762 beschrieben erfolgen.

Man erhält so eine TEA-Rohware mit einem Gehalt von ca. 80 Gew.-% TEA und ca. 20 Gew.-% DEA, sowie Nebenkomponenten, wie beispielsweise der oben beschriebenen Art, in geringen Mengen.

Die so erhaltene TEA-Rohware wird schließlich gemäß der EP-A-4015 mit einer wirksamen Menge von phosphoriger oder unterphosphoriger Säure (H₃PO₃ oder H₃PO₂) bzw. deren Verbindungen, bevorzugt phosphorige Säure, versetzt und im Vakuum rektifiziert.

Die phosphorige bzw. unterphosphorige Säure kann in monomerer oder gegebenenfalls polymerer Form, in wasserhaltiger Form (Hydrate), als Anlagerungsverbindung, oder Salz, wie beispielsweise Dinatriumhydrogenphosphit Na₂HPO₃, zugegeben werden. Auch Verbindungen der phosphorigen oder unterphosphorigen Säure, wie Ester, sind geeignet.

Die Menge an den zugesetzten Phosphorverbindungen beträgt in der Regel mindestens 0,01 Gew.-%, bevorzugt 0,01 bis 2 Gew.-%, besonders bevorzugt 0,02 bis 1,0 Gew.-%, ganz besonders bevorzugt 0,02 bis 0,1 Gew.-%, bezogen auf die eingesetzte Menge an Roh-TEA; die Wirkung tritt jedoch auch bei größeren Mengen ein.

Die Rektifikation erfolgt vorzugsweise kontinuierlich und bei einem Druck kleiner 10 mbar (10 hPa), beispielsweise ca. 1 bis 2 mbar, wobei Leichtsiederanteile über Kopf abgezogen werden. Man erhält so im Seitenabzug ein reines TEA.

Das erfindungsgemäße Verfahren liefert ein Triethanolamin (TEA) mit einer Reinheit größer 99 %, bevorzugt größer 99,4 %, das direkt nach der Rektifikation eine APHA-Farbzahl von 0 bis 10, insbesondere von 0 bis kleiner 6, ganz besonders von 0 bis 5, aufweist und auch nach einer Lagerzeit von mindestens 6 Monaten in einem geschlossenen Gebinde unter Lichtausschluss bei Temperaturen von 10 bis 30°C eine APHA-Farbzahl von kleiner 50, insbesondere von kleiner 40, ganz besonders von kleiner 35, aufweist.

Das Produkt des erfindungsgemäßen Verfahrens zeigt weder direkt nach der Rektifikation noch nach einer Lagerzeit von mindestens 6 Monaten (Lagerbedingungen wie oben) eine Rosaverfärbung beim eingangs genannten "Säureneutralisationstest".

Das erfindungsgemäße Verfahrensprodukt weist nach einer solchen Säurebehandlung, die wie am Ende des Beispiels beschrieben innerhalb von ca. 0,5 bis 1 Stunde nach dessen Erhalt ausgeführt wird, einen Wert für die Maßzahl a* nach dem CIE-Lab-System von kleiner/gleich 10, insbesondere von kleiner 7, ganz besonders von kleiner 3, auf.

### Beispiel 1

2 300 kg DEA/TEA-Rohgemisch (Gehalt: ca. 75 bis 80 Gew.-% DEA und ca. 25 bis 20 Gew.-% TEA), das nach destillativer Abtrennung des MEA-Anteils bei 30 bis 100 mbar aus einem Roh-Gemisch der Ethanolamine MEA, DEA und TEA, hergestellt nach Nr. 5 der Beispiele 1 und 2 der EP-A-673 920 aus wässrigem Ammoniak und Ethylenoxid unter Druck, erhalten wurde, wurde in einer 1. Stufe mit 330 kg Ethylenoxid in einem Rohrreaktor (20 m² Innenfläche) bei ca. 145°C in exothermer Reaktion und anschließend in einer 2. Stufe ebenfalls in einem Rohrreaktor (3,8 m² Innenfläche) mit weiteren 165 kg Ethylenoxid bei ca. 165°C weiter umgesetzt.

In einem weiteren Rohrreaktor (3,8 m² Innenfläche), der 3. Umsetzungsstufe, wurde die Reaktion zu Ende geführt, das Reaktionsgemisch dabei auf ca. 140°C abgekühlt und direkt in die Vorlage einer sich anschließenden kontinuierlichen, fraktionierenden Rektifikation gefahren oder alternativ weiter auf 80°C abgekühlt, zwischengelagert und dann erst über die beschriebene Destillationsvorlage in die kontinuierliche Rektifikation geleitet.

1 600 kg auf diese Weise erhaltenes Roh-TEA (Zusammensetzung: ca. 80 Gew.-% TEA, ca. 20 Gew.-% DEA und weniger als ca. 1 Gew.-% Nebenkomponenten, wie beispielsweise (2-(2-Hydroxyethoxy)ethyl)-di-(2-hydroxyethyl)-amin, wurden nach Zudosierung von ca. 0.56 kg phosphoriger Säure (H₃PO₃) (0,035 Gew.-% berechnet auf eingesetzte Menge an Roh-TEA) auf ca. 150 bis 180°C vorerhitzt in eine kontinuierlich betriebene Rektifikationskolonne gefahren.

Die Rektifikationskolonne war mit einem Umlaufverdampfer ausgestattet.

Bei einem Vakuum von 2 mbar (2 hPa) wurden über Kopf der Kolonne ca. 500 kg Leichtsiederanteile (Gehalt: ca. 70 Gew.-% DEA und ca. 30 Gew.-% TEA) abgezogen.

Über den Seitenabzug der Kolonne wurden ca. 900 kg farbloses reines TEA (Farbzahl: 0 - 5 APHA) mit einem Gehalt von 99,5 bis 99,7 % (GC) gewonnen.

Aus dem Kolonnensumpf wurden über einen nachgeschalteten Dünnschichtverdampfer bei einem Vakuum von ca. 1,5 bis 2 mbar (1,5 bis 2 hPa) ca. 200 kg Rückstand (Zusammensetzung: ca. 90 bis 95 Gew.-% TEA; Rest: Hochsieder, wie beispielsweise (2-(2-Hydroxyethoxy)ethyl)-di-(2-hydroxyethyl)-amin, abgetrennt. Das Kopfdestillat des Dünnschichtverdampfers wurde in den Sumpf der TEA-Rektifikationskolonne zurückgefahren.

Das auf diese Weise hergestellte TEA besaß nach halbjähriger Lagerzeit in einem geschlossenen Gebinde unter Lichtausschluss bei Temperaturen von 10 bis 30°C eine Farbzahl von 30 APHA, eine minimale Verfärbung, die das Auge gerade als zarte Gelbfärbung wahrzunehmen beginnt.

Die Messung der APHA-Farbzahlen erfolgte nach DIN-ISO 6271.

Das auf diese Weise hergestellte TEA färbte sich nach einer Säurebehandlung, die wie unten beschrieben ausgeführt wurde, augenscheinlich nicht rosa, auch nicht nach Durchführung der Säurebehandlung nachdem das TEA 6 Monate unter den o.g. Bedingungen gelagert worden war. ("Säureneutralisationstest").

Das auf diese Weise hergestellte TEA wies nach einer Säurebehandlung, die wie unten beschrieben innerhalb von 0,5 bis 1 Stunde nach dessen Erhalt ausgeführt wurde, einen Wert für die Maßzahl a* nach dem CIE-Lab-System von kleiner 3 auf.

Zur Beurteilung der Farbqualität des hergestellten TEAs wurde dieses zunächst wie folgt beschrieben mit einer Säure behandelt und die Maßzahl a* nach dem CIE-Lab-System bestimmt:

35 g einer Probe reines TEA, 7,5 g 1,2-Propandiol und 6,0 g 85%ige Phosphorsäure wurden durch Rühren gut vermischt und im Wärmeschrank 20 Min. auf 100°C erhitzt. Anschließend wurden für diese so behandelte TEA-Probe in einer spektralen Farbmessung die Maßzahlen L*, a* und b* des CIE-Lab-Systems nach Judd und Hunter (CIE = Comission International d'Eclairage, Paris) in einem LICO 200-Gerät der Firma Dr. Lange unter Verwendung einer 5 cm-Küvette ermittelt.

Die Maßzahl a* beschreibt hierbei den Rotanteil der Probe.

### Vergleichsbeispiel

2 300 kg DEA/TEA-Rohgemisch (Gehalt: ca. 75 bis 80 Gew.-% DEA und ca. 25 bis 20 Gew.-% TEA), das wie im obigen Beispiel beschrieben aus wässrigem Ammoniak und Ethylenoxid erhalten wurde, wurde nach Abtrennung von MEA kontinuierlich fraktionierend bei 10 bis 15 mbar unter Abtrennung eines Hauptteils des DEAs rektifiziert.

1700 kg auf diese Weise erhaltenes Roh-TEA (Zusammensetzung: ca. 80 Gew.-% TEA, ca. 20 Gew.-% DEA und weniger als ca. 1 Gew.-% Nebenkomponenten, wie beispielsweise (2-(2-Hydroxyethoxy)ethyl)-di-(2-hydroxyethyl)-amin, wurden nach Zudosierung von ca. 0,6 kg phosphoriger Säure (H₃PO₃) (0,035 Gew.-% berechnet auf eingesetzte Menge an Roh-TEA) auf ca. 150 bis 180°C vorerhitzt und in die Vorlage einer kontinuierlich betriebenen Rektifikationskolonne gefahren.

Die Rektifikationskolonne war mit einem Umlaufverdampfer ausgestattet.

Bei einem Vakuum von 2 mbar (2 hPa) wurden über Kopf der Kolonne ca. 600 kg Leichtsiederanteile (Gehalt: ca. 70 Gew.-% DEA und ca. 30 Gew.-% TEA) abgezogen.

Über den Seitenabzug der Kolonne wurden ca. 900 kg farbloses reines TEA (Farbzahl: 3 APHA) mit einem Gehalt von 99,5 bis 99,7 % (GC) gewonnen.

Aus dem Kolonnensumpf wurden über einen nachgeschalteten Dünnschichtverdampfer bei einem Vakuum von ca. 1,5 bis 2 mbar (1,5 bis 2 hPa) ca. 200 kg Rückstand (Zusammensetzung: ca. 90 - 95 Gew.-% TEA; Rest: Hochsieder, wie beispielsweise (2-(2-Hydroxyethoxy)ethyl)-di-(2-hydroxyethyl)-amin, abgetrennt. Das Kopfdestillat des Dünnschichtverdampfers wurde in den Sumpf der TEA-Rektifikationskolonne zurückgefahren.

Das auf diese Weise hergestellte TEA besaß nach halbjähriger Lagerzeit in einen geschlossenen Gebinde unter Lichtausschluss bei Temperaturen von 10 bis 30°C eine Farbzahl von größer 50 APHA, was als deutliche Gelbfärbung wahrzunehmen war.

Das auf diese Weise hergestellte TEA wies nach einer Säurebehandlung, die wie oben beschrieben innerhalb von 0,5 bis 1 Stunde nach dessen Erhalt ausgeführt wurde, einen Wert für die Maßzahl a* nach dem CIE-Lab-System von größer 10 auf.

### Beispiel 2

2 300 kg DEA/TEA-Rohgemisch (Gehalt: ca. 75 bis 80 Gew.-% DEA und ca. 25 bis 20 Gew.-% TEA), das wie in Beispiel 1 beschrieben erhalten wurde, wurde in einer 1. Stufe mit 435 kg Ethylenoxid in einem Rohrreaktor (20 m² Innenfläche) bei ca. 150°C in exothermer Reaktion und anschließend in einer 2. Stufe ebenfalls in einem Rohrreaktor (3,8 m² Innenfläche) mit weiteren 220 kg Ethylenoxid bei ca. 170°C weiter umgesetzt.

Das weitere Vorgehen erfolgte wie im Beispiel 1 beschrieben.

Das auf diese Weise hergestellte TEA färbte sich nach einer Säurebehandlung, die wie im Beispiel 1 beschrieben ausgeführt wurde, augenscheinlich nicht rosa, auch nicht nach Durchführung der Säurebehandlung, nachdem das TEA 6 Monate unter den im Beispiel 1 genannten Bedingungen gelagert worden war ("Säureneutralisationstest").

Das auf diese Weise hergestellte TEA wies nach einer Säurebehandlung, die wie im Beispiel 1 beschrieben erfolgte und innerhalb von 0,5 bis 1 Stunde nach dessen Erhalt ausgeführt wurde, einen Wert für die Maßzahl a* nach dem CIE-Lab-System von kleiner 3 auf.

### Beispiel 3

2 300 kg DEA/TEA-Rohgemisch (Gehalt: ca. 75 bis 80 Gew.-% DEA und ca. 25 bis 20 Gew.-% TEA), das wie in Beispiel 1 beschrieben erhalten wurde, wurde mit der Gesamtmenge von 495 kg Ethylenoxid nach Beispiel 1 in nur einer Stufe (Rohrreaktor 20 m² Innenfläche) bei 160°C umgesetzt, der 2. Rohrreaktor (3,8 m² Innenfläche) durchfahren und im 3. Rohrreaktor (3,8 m² Innenfläche) zu Ende geführt.

Die weitere Aufarbeitung erfolgte gemäß Beispiel 1.

Das auf diese Weise hergestellte TEA hatte die gleichen Eigenschaften wie im Beispiel 1 ausgeführt.

### Beispiel 4

Eingesetzt wurden 2 300 kg DEA/TEA-Rohgemisch entsprechend Beispiel 1, jedoch erfolgte die Zugabe von 330 kg Ethylenoxid in der 1. Stufe bei ca. 160°C und der restlichen 165 kg Ethylenoxid in der 2. Stufe bei ca. 180°C.

Das auf diese Weise hergestellte TEA hatte die gleichen Eigenschaften wie im Beispiel 1 ausgeführt.

### Beispiel 5

1 600 kg durch weitere Ethoxylierung von DEA/TEA-Rohgemisch gemäß Beispiel 1 hergestelltes Roh-TEA (Zusammensetzung: ca. 80 Gew.-% TEA, ca. 20 Gew.-% DEA und weniger als ca. 1 Gew.-% Nebenkomponenten, wie beispielsweise 2-(2-Hydroxyethoxy)ethyl-di-(2-hydroxyethyl)-amin, wurden nach Zudosierung von ca. 4,8 kg phosphoriger Säure (H₃PO₃) (0,3 Gew.-% berechnet auf eingesetzte Menge an Roh-TEA) auf ca. 150 bis 180°C vorerhitzt in eine kontinuierlich betriebene Rektifizierkolonne gefahren.

Das weitere Vorgehen entsprach dem Beispiel 1.

Das auf diese Weise hergestellte TEA hatte die gleichen Eigenschaften wie bei der Herstellung nach Beispiel 1.

## Patentansprüche

1. Verfahren zur Reinigung von Triethanolamin, hergestellt durch Umsetzung von wässrigem Ammoniak mit Ethylenoxid in flüssiger Phase unter Druck und bei erhöhter Temperatur, **dadurch gekennzeichnet, dass** man vom Umsetzungsprodukt überschüssiges Ammoniak, Wasser und Monoethanolamin abtrennt, das so erhaltene Rohprodukt mit Ethylenoxid bei Temperaturen von 110 bis 180 °C umsetzt und anschließend in Gegenwart von phosphoriger oder unterphosphoriger Säure oder deren Verbindungen rektifiziert.

2. Verfahren nach Anspruch 1, **dadurch gekennzeichnet, dass** man das nach der Abtrennung von überschüssigem Ammoniak, Wasser und Monoethanolamin erhaltene Rohprodukt mit 0,6 bis 1,2 Mol Ethylenoxid pro Grammatom an Stickstoff gebundenen Wasserstoff im Rohprodukt umsetzt.

3. Verfahren nach den Ansprüchen 1 und 2, **dadurch gekennzeichnet, dass** nach der Abtrennung von überschüssigem Ammoniak, Wasser und Monoethanolamin die Umsetzung mit Ethylenoxid mehrstufig erfolgt.

4. Verfahren nach den Ansprüchen 1 bis 3, **dadurch gekennzeichnet, dass** man in Gegenwart von mindestens 0,01 Gew.-%, bezogen auf die Menge an Roh-Triethanolamin, von phosphoriger oder unterphosphoriger Säure oder deren Verbindungen rektifiziert.

5. Verfahren nach den Ansprüchen 1 bis 4, **dadurch gekennzeichnet, dass** die Rektifikation in Gegenwart von phosphoriger Säure erfolgt.

6. Verfahren nach den Ansprüchen 1 bis 5, **dadurch gekennzeichnet, dass** das gereinigte Triethanolamin nach Säurebehandlung einen Wert für die Maßzahl a* nach dem CIE-Lab-System von kleiner/gleich 3 aufweist.

7. Verfahren nach den Ansprüchen 1 bis 6, **dadurch gekennzeichnet, dass** die APHA-Farbzahl (DIN-ISO 6271) des gereinigten Triethanolamins während einer sechsmonatigen Lagerung in einem geschlossenen Gebinde unter Lichtausschluss bei Temperaturen von 10 bis 30 °C kleiner 50 beträgt.

8. Verfahren nach Anspruch 7, **dadurch gekennzeichnet, dass** die APHA-Farbzahl des gereinigten Triethanolamins während einer sechsmonatigen Lagerung in einem geschlossenen Gebinde unter Lichtausschluss bei Temperaturen von 10 bis 30 °C kleiner 35 beträgt.

## Claims

1. A method of purifying triethanolamine prepared by reacting aqueous ammonia with ethylene oxide in the liquid phase under pressure and at elevated temperature, which comprises removing excess ammonia, water and monoethanolamine from the reaction product, reacting the resulting crude product with ethylene oxide at temperatures of from 110 to 180°C, and then rectifying the mixture in the presence of phosphorous or hypophosphorous acid or compounds thereof.

2. A method as claimed in claim 1, wherein the crude product resulting following removal of excess ammonia, water and monoethanolamine is reacted with from 0.6 to 1.2 mol of ethylene oxide per gram-atom of nitrogen-bonded hydrogen in the crude product.

3. A method as claimed in claims 1 and 2, wherein, following removal of excess ammonia, water and monoethanolamine, the reaction with ethylene oxide is carried out in a plurality of stages.

4. A method as claimed in any of claims 1 to 3, wherein the mixture is rectified in the presence of at least 0.01 % by weight, based on the amount of crude triethanolamine, of phosphorous or hypophosphorous acid or compounds thereof.

5. A method as claimed in any of claims 1 to 4, wherein the rectification is carried out in the presence of phosphorous acid.

6. A method as claimed in any of claims 1 to 5, wherein the purified triethanolamine has, after acid treatment, a value for the numerical measure a* according to the CIE Lab system of less than or equal to 3.

7. A method as claimed in any of claims 1 to 6, wherein the APHA color number (DIN-ISO 6271) of the purified triethanolamine during storage for 6 months in a sealed pack with the exclusion of light at temperatures of from 10 to 30°C is less than 50.

8. A method as claimed in claim 7, wherein the APHA color number of the purified triethanolamine during storage for 6 months in a sealed pack with the exclusion of light at temperatures of from 10 to 30°C is less than 35.

## Revendications

1. Procédé pour la purification de triéthanolamine, fabriquée par conversion d'ammoniaque aqueux avec de l'oxyde d'éthylène en phase liquide sous pression et à température accrue, **caractérisé en ce que** l'on sépare du produit de conversion l'eau, la monoéthanolamine et l'ammoniaque en excès, **en ce que** l'on convertit le produit brut ainsi obtenu avec de l'oxyde d'éthylène à des températures de 110 à 180°C et **en ce qu'**on le rectifie ensuite en présence d'acide phosphoreux ou hypophosphoreux ou de leurs dérivés.

2. Procédé selon la revendication 1, **caractérisé en ce que** l'on convertit le produit brut obtenu après la séparation de l'eau, la monoéthanolamine et l'ammoniaque en excès en excès avec 0,6 à 1,2 mole d'oxyde d'éthylène par mole d'atomes d'hydrogène lié à l'azote dans le produit brut.

3. Procédé selon les revendications 1 et 2, **caractérisé en ce que** l'on effectue la conversion avec l'oxyde d'éthylène en plusieurs étapes, après la séparation de l'eau, la monoéthanolamine et l'ammoniaque en excès en excès.

4. Procédé selon les revendications 1 à 3, **caractérisé en ce que** l'on effectue la rectification en présence d'au moins 0,01 % en poids, rapportés à la quantité de triéthanolamine brute, d'acide phosphoreux ou hypophosphoreux ou de leurs dérivés.

5. Procédé selon les revendications 1 à 4, **caractérisé en ce que** la rectification est effectuée en présence d'acide phosphoreux.

6. Procédé selon les revendications 1 à 5, **caractérisé en ce que** la triéthanolamine purifiée présente, après le traitement à l'acide, une valeur du coefficient a* selon le système de laboratoire CIE inférieure ou égale à 3.

7. Procédé selon les revendications 1 à 6, **caractérisé en ce que** le pouvoir colorant APHA (DIN-ISO 6271) de la triéthanolamine purifiée pendant un stockage de six mois dans un fût métallique fermé à l'abri de la lumière à des températures de 10 à 30°C est inférieur à 50.

8. Procédé selon la revendication 7, **caractérisé en ce que** le pouvoir colorant APHA de la triéthanolamine purifiée pendant un stockage de six mois dans un fût métallique fermé à l'abri de la lumière à des températures de 10 à 30°C est inférieur à 35.
